# EUROPÄISCHE PATENTANMELDUNG

(11) **EP 2 174 582 A1**
(43) Veröffentlichungstag der Anmeldung: **14.04.2010**
(21) Anmeldenummer: 09172001.1
(22) Anmeldetag: 01.10.2009
(51) Int. Cl.: A61B 1/00, A61B 1/005, A61B 1/01, A61B 1/31

(54) **Medizintechnischer, elastischer Polymerschlauch und Verfahren zu seiner Herstellung**

(30) Priorität: 09.10.2008 DE 102008042718
(71) Anmelder: INVENDO MEDICAL GMBH, 69469 Weinheim (DE)
(72) Erfinder: Bob, Konstantin, 69469, Weinheim (DE); Dillinger, Ilona, 69469, Weinheim (DE)
(74) Vertreter: Winter, Brandl, Fürniss, Hübner Röss, Kaiser, Polte Partnerschaft Patent- und Rechtsanwaltskanzlei

(57) **Zusammenfassung**

Es wird ein medizintechnischer, elastischer Polymerschlauch aus thermoplastischem Kunststoff oder aus thermoplastischen Kunststoffen, ein Verfahren zur Herstellung eines medizintechnischen, elastischen Polymerschlauchs aus thermoplastischem Kunststoff oder aus thermoplastischen Kunststoffen sowie ein Endoskop mit einem medizintechnischen, elastischen Polymerschlauch aus thermoplastischem Kunststoff oder aus thermoplastischen Kunststoffen zur Verfügung gestellt.

## Beschreibung

Die vorliegende Erfindung betrifft einen medizintechnischen, elastischen Polymerschlauch, ein Verfahren zur Herstellung eines medizintechnischen, elastischen Polymerschlauchs, sowie ein Endoskop, für welches ein medizintechnischer, elastischer Polymerschlauch eingesetzt wird.

Vorrichtungen zum Einführen eines medizinischen Endoskops in einen Körperkanal werden beispielsweise in der deutschen Patentanmeldung DE-A-39 25 484 beschrieben. Die darin beschriebenen Vorrichtungen erlauben es, dass ein Endoskop nicht mehr in den zu untersuchenden Körper hineingeschoben wird, sondern sich selber hineinbewegt. Zu diesem Zweck ist das Endoskop mit einem Eigenantrieb ausgestattet, der ein unkompliziertes und schnelles Einführen ermöglicht.

Als solch ein Eigenantrieb kann beispielsweise auch ein so genannter Stülpschlauch verwendet werden, in den der Endoskopschaft eingeführt ist. Beim Vortrieb des Endoskops treten unterschiedliche Relativbewegungen auf. Zum einen tritt eine Relativbewegung zwischen dem Endoskopschaft und dem Stülpschlauch auf, die miteinander in Gleitkontakt stehen. Zum anderen tritt auch eine Relativbewegung zwischen einem innen liegenden und einem außen liegenden Abschnitt des sich abwickelnden Stülpschlauchs auf.

Es gibt bei der gegenwärtigen Entwicklung derartiger Vorrichtungen das Bestreben, Schläuche herzustellen, die eine verminderte Reibung zwischen beispielsweise einem eingesetzten Stülpschlauch und einem Endoskopschaft, zwischen dem Stülpschlauch und einer Hülle des Endoskopschafts als auch zwischen einzelnen Abschnitten eines Stülpschlauchs erzielen können.

Bei den derzeit verwendeten Stülpschlauchvorrichtungen tritt jedoch das Problem auf, dass ab einer bestimmten Länge des Schlauchs der durch Reibung erzeugte Widerstand so hoch wird, dass der Stülpschlauch nicht weiter in den Patienten eingeführt werden kann. Weiterhin kann das Problem auftreten, dass der Stülpschlauch aufgrund der Kraftübertragung auf den Stülpschlauch Falten wirft und auch aus diesem Grunde ein weiterer Vortrieb nicht möglich ist.

Aufgabe der Erfindung ist es, einen verbesserten medizintechnischen Schlauch (im Folgenden auch "der Schlauch") bzw. ein verbessertes Verfahren zur Herstellung eines medizintechnischen Schlauchs sowie ein verbessertes Endoskop mit einem medizintechnischen Schlauch zur Verfügung zu stellen.

Die erfindungsgemäße Aufgabe wird durch die in den unabhängigen Ansprüchen definierten Merkmalskombinationen gelöst. Vorteilhafte Weiterbildungen sind den Unteransprüchen zu entnehmen.

Dabei ist es insbesondere eine Absicht der Erfindung, einen medizintechnischen elastischen Polymerschlauch und ein Verfahren zu seiner Herstellung bereitzustellen, der ein verbessertes mechanisches Verhalten beim Einführen in den Patienten aufweist. Insbesondere ist es eine Absicht der Erfindung, einen medizintechnischen Polymerschlauch, der in den Patienten über weitere Strecken als bisher eingeführt werden kann, sowie ein Verfahren zu seiner Herstellung bereitzustellen.

Die Erfindung bezieht sich gemäß einem ersten Aspekt auf einen medizintechnischen, elastischen Polymerschlauch aus thermoplastischem Kunststoff, wobei der Polymerschlauch mindestens zwei Schichten hat, die unterschiedliche Shore-Härten aufweisen.

Gemäß einem weiteren Aspekt stellt die vorliegende Erfindung zur Lösung der vorstehend genannten Aufgabe einen medizintechnischen, elastischen Polymerschlauch aus thermoplastischem Kunststoff zur Verfügung, wobei der Polymerschlauch eine oder mehrere Schichten hat und mindestens eine Oberfläche des Polymerschlauches funktionalisiert ist.

Nach einem weiteren Aspekt der Erfindung wird ein medizintechnischer, elastischer Polymerschlauch aus thermoplastischem Kunststoff zur Verfügung gestellt, der mindestens zwei Schichten hat, die unterschiedliche Shore-Härten aufweisen und in dem mindestens eine Oberfläche des Polymerschlauches funktionalisiert ist.

Daneben stellt die Erfindung ein Verfahren zur Herstellung derartiger medizintechnischer, elastischer Polymerschläuche sowie ein Endoskop mit medizintechnischen, elastischen Polymerschläuchen zur Verfügung.

Die erfindungsgemäßen Schläuche bestehen vorzugsweise aus mindestens zwei Schichten entweder aus einer Art von thermoplastischem Kunststoff oder aus mehreren Arten von thermoplastischen Kunstoffen. Der Schlauch ist bevorzugt durch Extrusion bzw. Co-Extrusion der mindestens zwei Schichten aus Kunststoffmaterial erhältlich. Das Kunststoffmaterial ist bevorzugt aus thermoplastischen Kunststoffen, vorzugsweise thermoplastischem Polyurethan (PUR), Polyvinylchlorid (PVC), Perfluorethylenpropylen-Copolymer (FEP), Polyamid (PA), Polyethylen (PE) und/oder Polytetrafluorethylen (PTFE) ausgewählt. In das/die Kunststoffmaterial(ien) kann ein Schmier- bzw. Gleitmittel eingearbeitet werden.

Das Material PVC weist den Vorteil auf, dass es sich aufgrund der Temperaturbeständigkeit (gegen Hitze und Kälte) mit allen in diesem technischen Bereich gängigen Sterilisationsverfahren (Dampfsterilisation, Ethylenoxidsterilisation, Strahlungssterilisation, Niedrig-Temperatur-Plasma-(Sterad-Plasma-) Sterilisation) sterilisieren lassen und problemlos gelagert werden kann. PVC besitzt zudem eine hohe Knickstabilität und hohe mechanische Belastbarkeit und einen geringen Abrieb. Schließlich hat PVC eine gute Verträglichkeit im medizinischen Bereich und wird daher auch für Allergiker empfohlen. PVC lässt sich wegen seines Dipolmoments der C-C1-Bindung im Polymer mittels Hochfrequenztechnik sicher mit sich selbst oder anderen Materialien verbinden.

Das Material PUR hat verschiedene physiko-chemische Vorteile und PUR stellt bereits ein weithin im medizinischen Bereich verwendetes und akzeptiertes Material dar.

Die Materialien PTFE und FEP haben den Vorteil, dass sie von sich aus schon über günstige Gleiteigenschaften verfügen und gegenüber Umwelteinflüssen sehr beständig sind.

Gemäß einer Weiterbildung ist mindestens eine Oberfläche des Schlauches mit negativ geladenen, positiv geladenen, polaren oder unpolaren Gruppen funktionalisiert, wobei es besonders bevorzugt ist, dass die Oberfläche hydrophil ist.

In einer Ausführungsform der Erfindung ist mindestens eine Außen- bzw. Oberfläche des Schlauches in einer derartigen Weise behandelt, dass die Oberfläche in einer gewünschten weise funktionalisiert wird. Insbesondere kann die Funktionalisierung in einer Weise erfolgen, dass die Hydrophilizität, Lipophilizität bzw. die Oberflächenspannung der Oberfläche eingestellt wird. Durch diese Einstellung der Hydrophilizität bzw. Lipophilizität ist es möglich, hydrophile oder lipophile Schmier- bzw. Gleitmittel über entsprechende Wechselwirkungen stärker an die Oberfläche zu binden und so die Reibung weiter zu vermindern. In einer besonders bevorzugten Ausführungsform können nach Einstellung einer erhöhten Hydrophilizität ("Hydrophilieren") Wasser oder wässrige Lösungen, wie z.B. physiologische Kochsalzlösung, oder wässrige Lösungen bzw. Dispersionen von Schmierstoffen wie Polyethylenglykol (PEG), Cellulose oder Algen-basierte Schmierstoffe, als Schmierbzw. Gleitmittel verwendet werden.

Bevorzugt erfolgt die Funktionalisierung entsprechend der in den Internationalen Patenanmeidungen WO-A-00/26180, WO-A-2006/075183 bzw. WO-A-2008/023170 beschriebenen Verfahren, wobei die Oberfläche eines Substrats mit einem Carben-Vorläufer kontaktiert wird, ein reaktives Carben-Intermediat aus dem Carben-Vorläufer erzeugt wird, so dass es mit dem Substrat zur Funktionalisierung der Oberfläche reagiert und das aktivierte Substrat durch das Einführen chemischer Gruppen mit gewünschten physikalischen bzw. chemischen Eigenschaften funktionalisiert wird. Das reaktive Carben-Intermediat wird bevorzugt aus dem Carben-Vorläufer unter Bedingungen erzeugt, die zu einer irreversiblen Bindung mit dem Substrat (d.h. der Schlauchoberfläche) führen. Dieses Vernetzen ("Curing") kann z.B. durch Erwärmen, Bestrahlen mit Licht und/oder Ultraschall erfolgen.

Bevorzugt kann die erhöhte Hydrophilizität der Oberflächen bzw. deren bessere Benetzbarkeit mit wässrigen Medien durch eine Oberflächenmodifikation durch die Einführung von geladenen Gruppen (z.B. Säuregruppen) oder die Einführung polarer Gruppen (z.B. Alkohole, Amine) erfolgen. Dadurch ist es möglich die Gleiteigenschaften der Oberfläche weiter zu verbessern. In gleichor Weise, d.h. durch Einführen entsprechender lipophiler Gruppen, kann auch die Lipophilizität und die Bindung lipophiler Schmier- bzw. Gleitmittel erhöht werden.

In einer bevorzugten Ausführungsform handelt es sich bei dem erfindungsgemäßen Schlauch um einen Stülpschlauch bzw. einen Endoskopschaft oder eine Endoskopschafthülle.

Es kann sich bei dem erfindungsgemäßen Schlauch auch um einen einschichtigen Schlauches handeln, der durch die vorher beschriebene Einstellung der Shore-Härte, die vorher beschriebene entsprechende Funktionalisierung mindestens einer Oberfläche und/oder die vorher beschriebene Beschichtung mindestens einer Oberfläche über verbesserte Eigenschaften verfügt. Im Falle eines einschichtigen Schlauches aus einem der vorher genannten thermoplastischen Kunststoffe ist die Shore-Härte bevorzugt im Bereich von 50 bis 65 und die Wanddicke des Schlauches ist bevorzugt kleiner als 1,6 mm.

Gemäß einer anderen Weiterbildung haben die Schichten des Polymerschlauchs eine unterschiedliche Dicke. Gemäß einer anderen Weiterbildung ist dabei die Shore-Härte mindestens einer Schicht höher als die Shore-Härte einer im Vergleich dazu dickeren Schicht. Bevorzugt ist dabei die Shore-Härte mindestens einer Schicht im Bereich von 50-65, bevorzugt von 55 bis 60 und die Shore-Härte mindestens einer Schicht im Bereich von 70-96.

Gemäß einer anderen Weiterbildung ist nach dem Ausstülpen eine dünnere und/oder härtere Schicht des Stülpschlauches die innere Oberfläche des Schlauches und eine dickere und/oder weichere Schicht die äußere Oberfläche des Schlauches, wobei letztere Schicht direkten Kontakt zum Patienten hat.

Dieser Aufbau des Schlauches erlaubt es, die gewünschte mechanische Festigkeit des Schlauchs zu erlagen. Insbesondere die Verwendung einer dünnen, harten Schicht in Kombination mit einer dicken, weichen Schicht mit der vorher angegebenen Reihenfolge bei Verwendung in einem Stülpschlauch besonders bevorzugt, da die harte dünne Schicht den Vorantrieb des Schlauchs unterstützt, während die die Darmwand auskleidende, weiche Schicht zu einem geringeren Biegeradius beiträgt und somit mechanische Irritationen im Patienten, z.B. der Darmwand, vermeidet bzw. reduziert.

Gemäß einer anderen Weiterbildung ist auf mindestens einer Oberfläche des Schlauchs eine Beschichtung aufgebracht, die bevorzugt aus Polyamid (PA), Polyethylen (PE) oder aus Perfluorethylenpropylen-Copolymer (FEP) ausgewählt ist. Für eine bessere Haftung dieser Beschichtung(en) kann, falls notwendig, eine Klebeschicht zwischen der Oberfläche und Beschichtung vorgesehen werden.

Das Aufbringen einer derartigen Beschichtung aus Substanzen mit guten Gleiteigenschaften wie PA, PE bzw. FEP setzt die unerwünschte Reibung weiter herunter.

Gemäß einer anderen Weiterbildung ist ein Schmier- bzw. Gleitmittel in das Schlauchmaterial compoundiert, wobei das Schmier- bzw. Gleitmittel bevorzugt aus Glycerin, Silikon- und/oder Paraffinöl, synthetischen Wachsen, Polyethylen, Polyethylenglykol (PEG) und anderen biokompatiblen Stoffen mit Schmiereigenschaften ausgewählt ist. Bevorzugt wird dabei während der Herstellung das Schmier- bzw. Gleitmittel, in das Schlauchmaterial compoundiert, d.h. in die geschmolzene Polymermasse vor der Extrusion gemischt wird (sog. "Compoundieren"). Dieses Einbringen des Schmier- bzw. Gleitmittels führt zu einer besonders vorteilhaften Schmierwirkung, d.h. der Verringerung der Reibung, wobei dieses Einbringen in das Material einen zusätzlichen Schritt des Aufbringens eines Schmier- bzw. Gleitmittels überflüssig machen kann.

Gemäß einer anderen Weiterbildung hat der Schlauch bevorzugt einen Innendurchmesser von 10,4 bis 12 mm und bevorzugt eine Wanddicke von 0,5 bis 1,6 mm.

Gemäß einer anderen Weiterbildung hat bevorzugt mindestens eine Schicht eine Dicke von 0,5 bis 1,3 mm bzw. eine Schicht eine Dicke von 0,02 bis 0,3 mm.

Gemäß einer anderen Weiterbildung ist der Polymerschlauch ein Stülpschlauch.

Gemäß einer bevorzugten Ausführungsform der Erfindung besteht der medizintechnische elastische Polymerschlauch aus mindestens zwei Schichten.

Im Folgenden bedeutet der Begriff "Innenschicht", dass es sich um einen Schicht des Schlauches handelt, die keine Berührung zur Umgebung hat, d.h. es befindet sich mindestens je eine weitere Schicht auf jeder Seite einer Innenschicht. Der Begriff "Außenschicht" bezieht sich auf Schichten mit direktem Kontakt zur Umgebung, wobei die "äußere Außenschicht" sich auf der Außenoberfläche des Schlauches befindet, während die "innere Außenschicht" sich auf der Innenoberfläche des Schlauches befindet, d.h. von der Schlauchwand umgeben ist.

Die Schichten des mehrschichtigen medizintechnischen Polymerschlauchs sind bevorzugt unterschiedlich dick, wobei es weiterhin bevorzugt ist, dass die Shore-Härte einer dünneren Schicht höher als die Shore-Härte einer dickeren Schicht des Schlauches ist.

Die Reihenfolge bzw. die Anzahl der Schichten wird jeweils gemäß der beabsichtigten Verwendung ausgewählt.

Im Falle eines Stülpschlauchs kann vor dem Ausstülpen des Schlauchs eine dünnere Schicht bzw. eine Schicht mit der höheren Härte eine äußere Außenschicht des Schlauches bilden. Im Fall eines zweischichtigen Aufbaus führt dies dazu, dass unmittelbar nach der Herstellung die dünne bzw. härtere Schicht die äußere Außenschicht des Schlauches (Außenoberfläche des Schlauches) bildet, und nach Ausstülpung des Schlauches sich die dünne bzw. härtere Schicht innen befindet und die dickere bzw. weichere Schicht im Kontakt zur Darmoberfläche des Patienten ist. Ein umgekehrter Aufbau ist jedoch ebenfalls möglich.

Bei einem Schlauch mit mehr als zwei Schichten können die beiden Außenschichten des Schlauches durch im Vergleich zu den anderen Schichten des Schlauches dünnere Schichten bzw. Schichten mit im Vergleich zu den anderen Schichten des Schlauches höherer Härte gebildet werden. Die dickere Schicht(en) bzw. die Schicht(en) mit im Vergleich zu den anderen Schichten des Schlauches geringerer Härte kann/können dabei eine Außenschicht bzw. eine Innenschicht des Schlauches bilden.

Die Einstellung der Shore-Härte der verschiedenen Schichten kann in einer bevorzugten Ausführungsform der Erfindung über die Zugabe von sogenannten Weichmachern erfolgen. Es ist aber auch möglich, die Shore-Härte der Schlauchschichten ohne die Verwendung von Weichmachern in Abhängigkeit vom verwendeten thermoplastischen Kunststoff durch entsprechende Auswahl der Herstellungsbedingungen des thermoplastischen Kunststoffs bzw. Kunststoffs einzustellen.

Bevorzugt sind die verwendeten Weichmacher toxikologisch unbedenklich und lösen sich nur in sehr geringem Maße aus dem Schlauchmaterial heraus, d.h. zeigen praktisch keine Migration aus dem Schlauchmaterial bzw. zwischen den verschiedenen Schichten des Schlauchs, so dass der Schlauch für längere Zeit (> 1 Jahr) gelagert werden kann und nur äußerst geringe Mengen an Weichmacher in den Patienten gelangen. Durch die kurze Verweildauer des Endoskopschlauchs in einem Patienten findet praktisch kein Übertritt eines Weichmachers in den Patienten statt.

Unterschiedliche Weichmacherkonzentrationen in verschiedenen Schlauchschichten können ggf. durch die Verwendung entsprechend unterschiedlicher Weichmacher so kompensiert werden, dass es keinen signifikanten Ausgleich durch Diffusion gibt. Zur Vermeidung eines Ausgleichs der Weichmacherkonzentration in den Wandschichten mit unterschiedlichen Shoro-Härten kann ggf. eine Trennschicht zwischen den verschiedenen Schichten des Schlauchs vorgesehen werden.

Besonders bevorzugt sind phthalatfreie Weichmacher, die Alternativen zu den bisher gebräuchlichen Weichmacher wie Dioktylphthalsäure (DOP) enthalten. Materialien mit derartigen Weichmachern werden auch als "NO DOP" Materialien bezeichnet. Derartige Weichmacher sind z.B. Diethylhexyterephthalat (DEHTP), Alykylsulfonsäureester (ÄSE), Acetyltributylcitrat (ATBC), Di-(2-ethylhexyl)adipat (DEHA; Synonym: DOA) und acetyliertes Ricinusölderivat, aber auch die in den Deutschen Patentschriften DE-T-60023 531, DE-A-101 41 250, DE-A-10 2004 029 135, DE-A-10 2004 036 202, DE-A-1 02 48 878 beschriebenen Weichmacher. Besonders bevorzugte Weichmacher sind Di-(isononyl)-cyclohexan-1,2-dicarboxylat (DINCH), Tri-(2-ethylhexyl)trimeliat (TEHTM) bzw. Tri-2-ethylhexyltrimelitsäureester (TOTM). Es können aber auch Kombinationen von einen oder mehreren dieser Weichmacher verwendet werden.

Die Menge des verwendeten Weichmachers hängt von der gewünschten Shore-Härte ab, wobei je höher der Anteil des Weichmachers ist, desto niedriger ist die Shore-Härte des Materials. Besonders bevorzugt können die Weichmacher in Konzentrationen bis zu 50 Gew.-% bezogen auf das Gesamtgewicht in dem verwendeten thermoplastischen Kunststoff enthalten sein.

Weitere Einzelheit zu verwendbaren Weichmachern und ihrer Konzentration, Verfahren zur Einstellung der Shore-Härte von Kunststoffen sowie geeigneten Additiven können z.B. dem "Taschenbuch der Kunststoff-Additive"; R. Gächter u. H. Müller (Hrsg.) 3.Ausgabe; München; Wien, Hanser 1989 bzw. "Kunststoff-Taschonbuch" 16. Ausgabe, Carl Hanser Verlag München entnommen werden.

Weiterhin können je nach Bedarf dem thermoplastischen Kunststoff Additive ausgewählt aus Antioxidantien, Füllstoffen, UV-Adsorbern, Stabilisatoren, Chelatbildnern und Pigmenten zugesetzt werden.

Gemäß einer anderen Weiterbildung ist mindestens eine Oberfläche des Schlauchs mit einem Schmier- bzw. Gleitmittel versehen. Vorzugsweise ist dabei das Schmier bzw. Gleitmittel bevorzugt aus Wasser bzw. auf Wasser basierenden Schmiermitteln, Polyethylenglykol (PEG), Cellulose oder auf Algen-basierenden Schmierstoffen, Glycerin, Paraffinöl, höheren Fettalkoholen, Metallseifen, Fettsäureglycerinestern, Polyethylen, synthetischen Wachsen, Agar-Agar, pflanzlichem Öl, einer Fett-Wachs-Mischung, (e)PTFE-Gleitlack, (e)PTFE-Pulver, Graphit, Talkum, verschiedenen Siliconüberzügen, Siliconöl, in Schmieröl dispergiertem Graphit- und/oder Teflon-Pulver sowie in Schmieröl dispergierten (e)PTFE- und/oder Glaskügelchen und anderen biokompatiblen Stoffen mit Schmiereigenschaften, oder einer Kombination von zwei oder mehreren von diesen, ausgewählt. Im Fall einer hydrophilen bzw. hydrophilisierten Oberfläche können Wasser bzw. auf Wasser basierende Schmiermittel verwendet werden. Im Fall einer nicht-funktionalisierten Oberfläche aus PVC kann Glycerin bzw. Paraffinöl und im Fall einer nicht-funktionalisierten Oberfläche aus PUR ein Siliconöl verwendet werden.

In einer weiteren besonders bevorzugten Ausführungsform der Erfindung wird ein medizintechnischer, elastischer Polymerschlauch aus thermoplastischem Kunststoff zur Verfügung gestellt, der mindestens zwei Schichten hat, wobei die Schichten sich zumindest in ihrer Shore-Härte wie vorher beschrieben voneinander unterscheiden und bei dem mindestens eine Außen- bzw. Oberfläche des Schlauches wie vorher beschrieben funktionalisiert ist.

Die vorher beschrieben Aspekte, Weiterbildungen bzw. Ausführungsformen sind frei miteinander kombinierbar, wobei jede Maßnahme an sich eine Verbesserung bringt, durch die Kombination sich jedoch Synergieeffekte ergeben.

Gemäß einer anderen Weiterbildung der Erfindung wird ein Verfahren zur Herstellung eines medizintechnischen, elastischen Polymerschlauchs aus thermoplastischem Kunststoff zur Verfügung gestellt , wobei der Polymerschlauch mehrschichtig aus thermoplastischen Kunststoffen mit unterschiedlicher Shore-Härte hergestellt wird, und/oder mindestens eine Oberfläche des Polymerschlauches einer die Reibung herabsetzenden Behandlung unterzogen wird.

Dabei sollte das Herstellungsverfahren für den medizintechnischen Polymerschlauch möglichst einfach sein, da ein derartiger Polymerschlauch ggf. ein Einwegartikel ist und somit in der Herstellung kostengünstig sein sollte. Zudem sollte sich das Verfahren möglichst leicht an unterschiedliche Schlauchmaterialien anpassen lassen.

Ferner ist es wünschenswert, dass das Verfahren einen Schlauch liefert, der sich mit weiteren Materialien wie etwa ergänzenden Schmier- bzw. Gleitmitteln leicht verhaften bzw. versehen lässt. Es ist eine weitere Aufgabe der Erfindung, einen entsprechenden Polymerschlauch als solchen bereitzustellen, dessen Gleiteigenschaften wie oben beschrieben verbessert sind, der kostengünstig ist und sich mit unterschiedlichen weiteren Schmier- bzw. Gleitmitteln hervorragend kombinieren lässt.

Gemäß einer anderen Weiterbildung werden in dem erfindungsgemäßen Verfahren ein oder mehrere thermoplastische Kunststoffe in mehreren Schichten coextrudiert.

Gemäß einer anderen Weiterbildung wird in dem erfindungsgemäßen Verfahren mindestens eine Schicht des Polymerschlauchs aus PVC, PUR, FEP, PA, PE oder PTFE hergestellt.

Gemäß einer anderen Weiterbildung wird in dem erfindungsgemäßen Verfahren mindestens eine Oberfläche des Schlauches funktionalisiert, wobei mindestens eine Oberfläche des Schlauches durch das Einbringen und/oder Aufbringen von negativ geladenen, positiv geladenen, polaren oder unpolaren Gruppen funktionalisiert wird, wobei bevorzugt mindestens eine Oberfläche des Schlauches hydrophiliert wird.

Gemäß einer anderen Weiterbildung erfolgt in dem erfindungsgemäßen Verfahren das Funktionalisieren mindestens einer Oberfläche des Schlauches bevorzugt durch die folgenden Schritte:
a) Kontaktieren der Schlauchoberfläche mit einem Carben-Vorläufer;
b) Erzeugen eines reaktiven Carben-Intermediats aus dem Carben-Vorläufer, so dass es mit dem Material der Oberfläche zur Funktionalisierung der Oberfläche reagiert; und
c) weiteres Funktionalisieren der erhaltenen aktivierten Oberfläche durch das Einführen chemischer Gruppen mit gewünschten physikalischen bzw. chemischen Eigenschaften.

Bevorzugt wird der Carben-Vorläufer durch Tauch- und/oder Sprühbeschichten aufgebracht.

Gemäß einer anderen Weiterbildung werden in dem erfindungsgemäßen Verfahren die Shore-Härten der Schichten des Polymerschlauchs und/oder die Dicke der Schichten unterschiedlich eingestellt, wobei bevorzugt die Shore-Härte mindestens einer Schicht höher als die Shore-Härte mindestens einer im Vergleich dazu dickeren Schicht eingestellt wird. Bevorzugt wird die Shore-Härte mindestens einer weicheren Schicht im Bereich von 50-65, bevorzugt von 55 bis 60, und mindestens einer härteren Schicht im Bereich von 70-96 eingestellt.

Gemäß einer anderen Weiterbildung wird in dem erfindungsgemäßen Verfahren auf mindestens eine Oberfläche des Schlauchs eine Beschichtung, die bevorzugt aus Polyamid (PA), Polyethylen (PE) oder aus Perfluorethylenpropylen-Copolymer (FEP) ausgewählt ist, aufgebracht, wobei ggf. eine Klebeschicht zwischen der Oberfläche und der Beschichtung vorgesehen wird, Die Beschichtung kann durch Extrusion, Kleben, Tauch- und/oder Sprühbeschichten aufgebracht werden.

Gemäß einer anderen Weiterbildung wird der Innendurchmesser des Schlauchs bevorzugt auf 10,4 bis 12mm eingestellt wird.

Gemäß einer anderen Weiterbildung wird die Wanddicke des Schlauchs auf 0,5 bis 1,6mm eingestellt.

Gemäß einer anderen Weiterbildung wird die Dicke mindestens einer Schicht auf von 0,5 bis 1,3mm eingestellt.

Gemäß einer anderen Weiterbildung wird die Dicke mindestens einer Schicht von 0,02 bis 0,3 mm eingestellt.

Gemäß einer anderen Weiterbildung wird ein Schmier- bzw. Gleitmittel in mindestens einen thermoplastischen Kunststoff compoundiert.

Gemäß einer anderen Weiterbildung wird beim Compoundieren das Schmierbzw. Gleitmittel aus Glycerin, Silikon- und/oder Paraffinöl, synthetischen Wachsen, Polyethylen, Polyethylenglykol (PEG) und anderen biokompatiblen Stoffen mit Schmiereigenschaften ausgewählt.

Gemäß einer anderen Weiterbildung wird auf mindestens einer Oberfläche des Schlauchs zusätzlich ein Schmier- bzw. Gleitmittel vorgesehen.

Die Ausformung des Kunststoffmaterials in Schlauchform bei der Herstellung des Schlauchs in dem erfindungsgemäßen Verfahren kann durch an sich bekannte Verfahren der Kunststoffverarbeitung erfolgen. Bevorzugt wird erfindungsgemäß ein Extrusionsvorgang, da hierbei dem Schlauch eventuell erwünschte spezielle Querschnittsgestalten und/oder Profile verliehen werden können. Dabei kann bei einem derartigen Extrusionsvorgang eine profilierte Oberfläche mit z.B. noppenförmigen Vorsprüngen auf wenigstens einer Schlauchoberfläche erzeugt werden. "Schlauchoberfläche^ meint hierbei sowohl die Außen- als auch Innenmantel)fläche des Schlauchs. Für die Herstellung des erfindungsgemäßen medizintechnischen Polymerschlauchs wird bevorzugt ein Extrusionsverfahren verwendet, wobei es besonders bevorzugt ist, dass die mindestens zwei Schichten des mehrschichtigen Schlauchs gemeinsam d.h. coextrudiert werden.

Bevorzugt wird durch die Coextrusion eine Haftung zwischen den einzelnen Schichten des Schlauchs vermittelt. Dies kann aber ebenfalls durch für die jeweiligen Kunststoffe geeignete Haftvermittler oder Oberflächenbehandlungen erfolgen. Im Falle von PVC ist aufgrund des Dipolmoments der C-CI-Bindung im PVC Polymer mittels Hochfrequenztechnik eine sichere Verbindung der Schichten möglich.

Bei der Herstellung kann ein .Schmier- bzw. Gleitmittel, bevorzugt Glycerin, Silikon- und/oder Paraffinöl, synthetische Wachse, Polyethylen, Polyethylenglykol (PEG) oder andere biokortipatible Schmierstoffe in das Schlauchmaterial compoundiert, d.h. in die geschmolzene Polymermasse vor der Extrusion gemischt werden (sog. "Compoundieren") . Dieses Einbringen des Schmier- bzw. Gleitmittels führt zu einer besonders vorteilhaften Schmierwirkung, d.h. der Verringerung der Reibung, wobei dieses Einbringen in das Material einen zusätzlichen Schritt des Aufbringens eines Schmier- bzw. Gleitmittels überflüssig machen kann.

Beim erfindungsgemäßen Herstellungsverfahren der erfindungsgemäßen Kunststoffschläuche kann entweder beim Extrusionsvorgang selber oder nach dem Extrusionsvorgang in einem gesonderten Schritt ein Profil, z.B. in der Form von Noppen auf mindestens einer Schlauchoberfläche erzeugt werden. Die beim Extrusionsvorgang erzeugte profilierte Oberfläche in Form einer Noppe ist bevorzugt beispielsweise ein kontinuierlicher, spiralförmig umlaufender Vorsprung.

In einer bevorzugten Ausführungsform der Erfindung ist mindestens eine der Oberfläche des Stülpschlauchs mit einer Beschichtung aus einem Polyolefin, bevorzugt Polyethylen (PE) versehen. Die hat den Vorteil, dass Oberflächen aus Polyolefinen, bevorzugt Polyethylen, eine gute Gleitfähigkeit ohne den Zusatz weiterer Schmier- bzw. Gleitmittel haben.

In einer weiteren bevorzugten Ausführungsform der Erfindung wird auf mindestens eine Oberfläche des Schlauchs eine Beschichtung aus einem Fluorelastomer aufgebracht, wobei ein Perfluorethylenpropylen-Copolymer (FEP) besonders bevorzugt ist.

In einer weiteren bevorzugten Ausführungsform der Erfindung wird auf mindestens eine Oberfläche des Schlauchs eine Beschichtung aus Polyamid (PA) aufgebracht.

Da Polyolefine, Fluorelastomere bzw. Polyamid möglicherweise nur eine geringe Eigenhaftung auf anderen thermoplastischen Kunststoffen aufweisen können, kann es im Fall der Verwendung von Polyolefinen und Fluorelastomeren bzw. Polyamid notwendig sein, die Haftung der durch die Verwendung von Haftvermittlern bzw. Klebern, z.B. niedermolekularen Polyolefinen mit Säuregruppen an einem Ende zu vermitteln bzw. zu erhöhen.

Auch hinsichtlich des Verfahrens der Erfindung sind die vorher beschrieben Aspekte, Weiterbildungen bzw. Ausführungsformen frei miteinander kombinierbar, wobei jede Maßnahme an sich eine Verbesserung bringt, durch die Kombination sich jedoch Synergieeffekte ergeben.

Gemäß der Erfindung wird ferner ein medizintechnischer Polymerschlauch bereitgestellt, der durch das erfindungsgemäße Verfahren erhältlich ist.

Die vorliegende Erfindung stellt zudem ein Endoskop bzw. Koloskop mit einem Stülpschlauchantrieb bereit, wobei das Endoskop (bzw. der Endoskopschaft oder eine darüber gestülpte Endoskopschafthülle) und/oder der Stülpschlauch bevorzugt aus dem vorher beschriebenen Kunststoffmaterial besteht.

Der Stülpschlauch wickelt sich dabei auf der Endoskopschafthülle ab. Dabei ist es des Weiteren bevorzugt, dass sich zwischen und/oder in den noppenförmigen Vorsprüngen ein Schmier- bzw. Gleitmittel befindet. Dabei ist es bevorzugt, dass die noppenförmigen Vorsprünge als perforierte Schmier- bzw. Gleitmittelreservoire ausgebildet sind. In einer bevorzugten Ausführungsform sind die noppenförmigen Vorsprünge durch Kugeln aus (e)PTFE oder Glas gebildet. Der bevorzugte Kugeldurchmesser für die Kugeln liegt im Bereich bis 0,2 mm. Der Einsatz derartiger Kugeln bewirkt eine weitergehende Verringerung der Reibung, welche sowohl bei einer Reibung Schlauch-auf-Schlauch als auch bei einer Reibung Schlauch-auf-Schaft deutlich verbesserte Gleiteigenschaften ergibt. Ein Grund für diese Verbesserung liegt in einer deutlich verkleinerten Auflagefläche, nämlich nur dem tangentialen Bereich der Kugeln. Zudem dienen die Kugeln als Abstandshalter, zwischen welche ein weiteres Schmier- bzw. Gleitmittel eingefüllt werden kann. Die Kugeln entsprechen diesbezüglich in ihrer Wirkung den oben erwähnten Noppen.

Insbesondere wenn der medizintechnische Polymerschlauch der vorliegenden Erfindung als Stülpschlauch eingesetzt wird, eignet sich ein wie vorstehend beschriebener thermoplastischer Kunststoff besonders gut als Grundsubstanz für diesen Schlauch, weil er vergleichsweise kostengünstig ist und leicht so bearbeitet werden kann, dass die angestrebte Verbesserung erzielt werden kann.

Kommt der medizintechnische Polymerschlauch der vorliegenden Erfindung als Endoskop-Schafthülle zum Einsatz, sind die vorher beschriebenen thermoplastischen Materialen sehr gut geeignet. Hiermit lässt sich eine gute Verbindung mit dem eigentlichen Schaft bei gleichzeitiger Reibungsverringerung gegenüber der Stülpschlauchaußenfläche erzielen.

In einer bevorzugten Ausführungsform wird der Stülpschlauch bzw. das Koloskop in einer lichtundurchlässigen Verpackung bereitgestellt, um eine vorschnelle Alterung der verwendeten Materialien zu verhindern.

Als zusätzliche Schmier- bzw. Gleitmittel, die auf die Schlauchoberfläche(n) aufgetragen werden, kommen die oben genannten Schmier- bzw. Gleitmittel sowie insbesondere nicht-hydrierte oder teilweise bis vollständig hydrierte pflanzliche Öle wie etwa Rapsöl und Sonnenblumenöl in Betracht. Besonders gut ist auch ein wachshaltiges Fett bzw. Fettgemisch geeignet, etwa ein Öl oder Ölgemisch, vorzugsweise eine Mischung aus pflanzlichem Öl und Wachs.

Es hat sich gezeigt, dass der Einsatz einer 50:50-Mischung aus vollständig hydriertem Rapsöl und hoch ölsäurehaltigem Sonnenblumenöl (so genanntes HO-Sonnenblumenöl), die einen Erstarrungspunkt im Bereich von bevorzugt 25 bis 50°C, mehr bevorzugt 30 bis 40°C und insbesondere bevorzugt 35 bis 39°C sowie einen Klarschmelzpunkt von bevorzugt 50 bis 60 und mehr bevorzugt 54 bis 56°C aufweist, sehr gute Gleiteigenschaften hat und in endoskopischen Anwendungen mit guter Verträglichkeit eingesetzt werden kann. Um die Haftung einer derartigen Mischung an dem Polymerschlauch (entweder dem Polymerschlauch als solchem oder dem Polymerschlauch mit noppenförmigen Vorsprüngen) noch weiter zu verbessern, ist der Mischung vorzugsweise eine gewisse Menge an Wachs beigefügt bzw. bereits enthalten, etwa in nicht-winterisiertem Öl (nicht-fraktioniertem Öl).

Ein oben beschriebenes Fett, Fettgemisch, Öl bzw. Ölgemisch kann auch in das Kunststoffmaterial selber eingebracht sein, vorzugsweise in das Kunststoffmaterial aus thermoplastischem Kunststoff, insbesondere jenem aus thermoplastischem Polyurethan.

Beim Extrudieren des Materials zur Ausbildung des erfindungsgemäßen Schlauchs kann dabei besonders gut eine auf der Schlauchoberfläche durchgängige, spiralförmig umlaufende Noppe (Vorsprung) ausgebildet werden.

Es ist ferner möglich, die Noppen in einem der Extrusion nachfolgenden gesonderten Schritt zu erzeugen, beispielsweise durch Schneiden, Stanzen, Prägen, Lasergravur oder dergleichen. Bei diesen Vorgängen wird in der Regel das sich zwischen den Noppen verbleibende Material abgetragen. Die Noppen können allerdings in dem gesonderten Schritt auch aus einer glatten Oberfläche heraus erzeugt werden, beispielsweise durch Aufschäumen spezieller Oberflächenbereiche, etwa durch lokales, z.B. punktförmiges, Erhitzen der Oberfläche (beispielsweise mit einem Laser).

Derartige noppenförmige Vorsprünge (bzw. Noppen), z.B. eine spiralförmige Noppe, auf der Oberfläche dienen als Abstandhalter auf der Schlauchoberfläche, zwischen welche ein Schmier- bzw. Gleitmittel eingefüllt werden kann.

Möglich ist auch, dass sich das Schmier- bzw. Gleitmittel alternativ oder ergänzend in den Noppen selber befindet, welche dann als Schmier- bzw. Gleitmittelreservoir dienen. Aus diesen Schmier- bzw. Gleitmittelreservoiren kann das Schmier- bzw. Gleitmittel beispielsweise bei Druckbeaufschlagung austreten.

Der durch das vorstehend beschriebene Verfahren hergestellte Schlauch ist bereits als solcher sehr gut für medizintechnische Anwendungen, insbesondere in der Endoskopie, geeignet, um eine weitere Verbesserung der Gleiteigenschaften zu erreichen, ist oftmals von Vorteil, ein weiteres Schmier- bzw. Gleitmittel auf die einer Reibung ausgesetzten Schlauchoberflächen aufzubringen.

Das in der vorliegenden Erfindung beschriebene Endoskop weist einen Stülpschlauchantrieb auf, wobei das Endoskop (d.h. der Endoskopschaft oder eine darüber gestülpte Endoskopschafthülle) und/oder der Stülpschlauch aus einem Kunststoffmaterial besteht, welches vorzugsweise aus den oben genannten ausgewählt ist.

Wie vorstehend erwähnt ist das erfindungsgemäße Verfahren insbesondere darauf ausgerichtet, einen Polymorschlauch herzustellen, der eine Schafthülle für ein Endoskop oder einen Stülpschlauch für ein Endoskop bildet. Entsprechend ist in die vorliegende Erfindung auch ein medizintechnischer Polymerschlauch eingeschlossen, welcher durch das erfindungsgemäße Verfahren erhältlich ist.

### Beispiele

Im Folgenden wird die Erfindung anhand von Beispielen erläutert, wobei diese Beispiele der Veranschaulichung dienen und nicht als beschränkend zu interpretieren sind.

### 1. Beispiel

Es werden folgende Varianten eines ein- bzw. mehrschichtigen Stülpschlauchs für ein Endoskop aus PVC durch Extrusion hergestellt:
a) Ein einschichtiger Schlauch aus PVC (Shore-Härte 55), einer Wanddicke von 0,9mm sowie einem Innendurchmesser von 10,5mm wird mittels eines Extruders extrudiert. Die unmittelbar nach der Extrusion entstehende innere Außenschicht bzw. Innenoberfläche (d.h. die Außenoberfläche nach Ausstülpen) wird mit einer Klebeschicht aus niedermolekularen Polyolefinen mit Säuregruppen an einem Ende versehen und eine Schicht aus PE in einer Dicke von 0,05mm aufgetragen. In einer weiteren Variante dieses Beispiels werden beide Außenschichten des Schlauchs mit PE beschichtet.
b) Ein zweischichtiger Schlauch aus PVC mit einer äußeren Schicht (Shore-Härte 90; Dicke 0,1mm), einer inneren Schicht (Shore-Härte 55; Dicke 0,8mm) und mit einer Wanddicke von 0,9mm sowie einem Innendurchmesser von 10,5mm wird mittels eines Extruders extrudiert. Die unmittelbar nach der Extrusion entstehende Innenoberfläche (d.h. die Außenoberfläche nach Ausstülpen) wird mit einer Klebeschicht aus niedermolekularen Polyolefinen mit Säuregruppen an einem Ende versehen und eine Schicht aus PE in einer Dicke von 0,05rnm aufgetragen. In einer weiteren Variante dieses Beispiels werden beide Außenschichten des Schlauchs mit PE beschichtet.

### 2. Beispiel

Es werden Schläuche aus PVC gemäß den Bedingungen des Beispiels 1 hergestellt, wobei statt einer PE-Beschichtung eine Beschichtung aus FEP in einer Dicke von 0,imm auf eine oder beide Außenschichten (Oberflächen) des Stülpschlauchs aufgebracht wird.

### 3. Beispiel

Es werden zwei Varianten von Schläuchen aus PVC gemäß den Bedingungen des Beispiels 1 hergestellt, wobei statt einer PE-Beschichtung eine Funktionalisierung der Oberfläche mit hydrophilen Gruppen unter Verwendung von Carben-Vorläufern erfolgte.

### 4. Beispiel

Es wird ein zweischichtiger coextrudierter Schlauch mit einer Schicht aus PVC (Shore-Härte 60) und mit einer Schicht aus PUP\ ^Shore-Härte 70) entsprechend den Bedingungen und Varianten der Beispiele 1 bis 3 in einer Weise extrudiert, dass die PVC-Schicht unmittelbar nach der Extrusion die äußere Schicht des Schlauchs bildet. Die beiden Außenschichten des extrudierten zweischichtigen Schlauches wurden entsprechend den Beispielen 1 bis 3 beschichtet bzw. funktionalisiert.

### 5. Beispiel

Ein einschichtiger Schlauch aus PTFE (Shore-Härte 65) und einer Wanddicke von 0,8mm sowie einem Innendurchmesser von 10,5mm wird mittels eines Extruders extrudiert.

### 6. Beispiel

Es werden folgende Varianten eines ein- bzw. mehrschichtigen Stülpschlauchs für ein Endoskop aus PUR durch Extrusion hergestellt:
a) Ein einschichtiger Schlauch aus PUR (Shore-Härte 65) und einer Wanddicke von 0,7mm sowie einem Innendurchirtesser von 11,1mm wird mittels eines Extruders extrudiert. In das PUR wird ein Schmier- bzw. Gleitmittel (z.B. Glycerin, Paraffinöl, Silikonöl, synthetische Wachse, Polyethylen, Polyethylenglycol (PEG)) compoundiert, wobei eine glatte, ölig-schmierige Oberfläche erzeugt wird, bei der man auf eine Beschichtung, evtl. auch auf den Zusatz eines Schmierbzw. Gleitmittels verzichten kann.
b) Ein zweischichtiger Schlauch aus PUR mit einer äußeren Schicht (Shore-Härte 90; Dicke 0,12mm), und mit einer inneren Schicht (Shore-Härte 50; Dicke 0,8mm) und einer Wanddicke von 0,92mm sowie einem Innendurchmesser von 11,1 mm wird mittels eines Extruders extrudiert. Die unmittelbar nach der Extrusion entstehende Innenoberfläche (d.h. die Außenoberfläche mit Kontakt zum Patienten nach Ausstülpen) wird einer Beschichtung mit PE bzw. FEP wie in den vorgehenden Beispiel oder einer Funktionalisierung der Oberfläche mit hydrophilen Gruppen unter Verwendung von Carben-Vonäufern unterzogen.

## Patentansprüche

1. Medizintechnischer, elastischer Polymerschlauch aus thermoplastischem Kunststoff oder aus thermoplastischen Kunststoffen, wobei
a) der Polymerschlauch mindestens zwei Schichten hat, die unterschiedliche Shore-Härten aufweisen; und/oder
b) der Polymerschlauch eine oder mehrere Schichten hat und mindestens eine Oberfläche des Polymerschlauches funktionalisiert ist.

2. Medizintechnischer, elastischer Polymerschlauch nach einem der vorhergehenden Ansprüche, wobei mindestens eine Oberfläche des Schlauches hydrophil ist.

3. Medizintechnischer, elastischer Polymerschlauch nach einem der vorhergehenden Ansprüche, wobei die Schichten eine unterschiedliche Dicke haben.

4. Medizintechnischer, elastischer Polymerschlauch nach einem der vorhergehenden Ansprüche, wobei die Shore-Härte mindestens einer Schicht im Bereich von 50-65, bevorzugt von 55 bis 60, ist.

5. Medizintechnischer, elastischer Polymerschlauch nach einem der vorhergehenden Ansprüche, wobei die Shore-Härte mindestens einer Schicht im Bereich von 70-96 ist.

6. Medizintechnischer, elastischer Polymerschlauch nach einem der vorhergehenden Ansprüche, wobei
a) in einem Schlauch mit zwei Schichten eine dünnere und/oder härtere Schicht des Schlauches die innere Außenschicht des Schlauches und eine dickere und/oder weichere Schicht die äußere Außenschicht des Schlauches ist; oder
b) bei einem Schlauch mit drei Schichten je eine dünnere und/oder härtere Schicht des Schlauches die innere und äußere Außenschicht des Schlauches ist und mindestens eine dickere und/odor weichere Schicht eine Innenschicht des Schlauches ist.

7. Medizintechnischer, elastischer Polymerschlauch nach einem der vorhergehenden Ansprüche, wobei ein Schmier- bzw. Gleitmittel in mindestens einen thermoplastischen Kunststoff compoundiert ist.

8. Medizintechnischer, elastischer Polymerschlauch nach Anspruch 7 wobei das Schmier- bzw. Gleitmittel aus Glycerin, Silikon- und/oder Paraffinöl, synthetischen Wachsen, Polyethylen, Polyethylenglykol (PEG) und anderen biokompatiblen Stoffen mit Schmier- bzw. Gleiteigenschaften oder einer Kombination von zwei oder mehreren von diesen ausgewählt ist.

9. Medizintechnischer, elastischer Polymerschlauch nach einem der vorhergehenden Ansprüche, wobei mindestens eine Oberfläche des Schlauchs mit einem Schmier- bzw. Gleitmittel versehen ist.

10. Medizintechnischer, elastischer Polymerschlauch nach Anspruch 9, wobei das Schmier- bzw. Gleitmittel aus Wasser bzw. auf Wasser basierenden Schmiermitteln, Polyethylenglykol (PEG), Cellulose oder auf Algenbasierenden Schmierstoffen, Glycerin, Paraffinöl, höheren Fettalkoholen, Metallseifen, Fettsäureglycerinestern, Polyethylen, synthetischen Wachsen, Agar-Agar, pflanzlichem Öl, einer Fett-Wachs-Mischung, (e)PTFE-Gleitlack, (e)PTFE-Pulver, Graphit, Talkum, verschiedenen Siliconüberzügen, Siliconöl, in Schmieröl dispergiertem Graphit- und/oder Teflon-Pulver sowie in Schmieröl dispergierten (e)PTFE- und/oder Glaskügelchen und anderen biokompatiblen Stoffen mit Schmier- bzw. Gleiteigenschaften, oder einer Kombination von zwei oder mehreren von diesen, ausgewählt ist.

11. Medizintechnischer, elastischer Polymerschlauch aus thermoplastischem Kunststoff, wobei
a) der Polymerschlauch aus einer Schicht besteht, die eine Shore-Härte von 50-65 aufweist; und/oder
b) mindestens eine Oberfläche des Polymerschlauches funktionalisiert ist.

12. Medizintechnischer, elastischer Polymerschlauch nach Anspruch 11, wobei die Wandstärke kleiner als 1,6 mm ist.

13. Verfahren zur Herstellung eines medizintechnischen, elastischen Polymerschlauchs aus thermoplastischem Kunststoff oder aus thermoplastischen Kunststoffen, wobei
a) der Polymerschlauch mehrschichtig aus thermoplastischen Kunststoffen mit unterschiedlicher Shore-Härte hergestellt wird, und/oder
b) mindestens eine Oberfläche des Polymerschlauches mit einer oder mehreren Schichten aus thermoplastischem Kunststoff funktionalisiert wird.

14. Verfahren nach einem der vorhergehenden Ansprüche, wobei die Shore-Härte der Schichten unterschiedlich eingestellt wird.

15. Verfahren nach einem der vorhergehenden Ansprüche, wobei die Dicke der Schichten unterschiedlich eingestellt wird.

16. Verfahren nach Anspruch 13, wobei ein auf eine Schlauchoberfläche aufgebrachtes Schmier- bzw. Gleitmittel aus Wasser bzw. auf Wasser basierenden Schmiermitteln, Polyethylenglykol (PEG), Cellulose oder auf Algen-basierenden Schmierstoffen, Glycerin, Paraffinöl, höheren Fettalkoholen, Metallseifen, Fettsäureglycerinestern, Polyethylen, synthetischen Wachsen, Agar-Agar, pflanzlichem Öl, einer Fett-Wachs -Mischung, (e)PTFE-Gleitlack, (e)PTFE-Pulver, Graphit, Talkum, verschiedenen Siliconüberzügen, Siliconöl, in Schmieröl dispergiertem Graphit- und/oder Teflon-Pulver sowie in Schmieröl dispergierten (e)PTFE- und/oder Glaskügelchen und anderen biokompatiblen Stoffen mit Schmier- bzw.
Gleiteigenschaften, oder einer Kombination von zwei oder mehreren von diesen, ausgewählt wird.

17. Endoskop mit einem Polymerschlauch nach einem der Ansprüche 1 bis 12 oder mit einem Polymerschlauch, der nach einem Verfahren der Ansprüche 13 bis 16 hergestellt ist.
